# EUROPEAN PATENT SPECIFICATION

(11) **EP 3 590 478 B1**
(45) Date of publication and mention of the grant of the patent: **07.06.2023**
(21) Application number: 19183893.7
(22) Date of filing: 02.07.2019
(51) Int. Cl.: A61F 9/00

(54) **EYE DROP APPLICATION**
AUGENTROPFENANWENDUNG
APPLICATION DE GOUTTES OPHTALMIQUES

(30) Priority: 06.07.2018 GB 201811130
(43) Date of publication of application: 08.01.2020
(73) Proprietor: Gokani, Bhavesh, Watford, Hertfordshire WD25 0JL (GB)
(72) Inventor: Gokani, Bhavesh, Watford, Hertfordshire WD25 0JL (GB)
(74) Representative: CSY Herts

(56) References cited:
- EP-A1- 0 347 084
- US-A- 4 733 802
- US-A1- 2009 281 508
- "Autodrop Eye Drop Guide", , 14 March 2015 (2015-03-14), XP002795612, Retrieved from the Internet: URL:https://www.arthritissupplies.com/auto drop-eye-drop-guide.html [retrieved on 2019-11-13]

## Description

### Field of the Invention

This invention relates to the application of eye drops and has for its object the use of an eye drop applicator.

In US Patent Specification No. 4,733,802 there is disclosed an eye drop applicator that comprises a body of generally inverted bowl shape formed of an opaque material with an opening in the top of the inverted bowl into which the top of a bottle of eye drops can be inserted.

Another form of eye drop applicator is described in EP 0 347 084 A1.

### Summary of the Invention

According to the present invention there is provided the use of an eye drop applicator according to appended independent claim 1.

The body may be formed of a resiliently deformable plastic material. If, however, the applicator is intended to be a single use device, it may be formed of paper or from a recycled and disposable pulp material.

The circumference of the body of bowl shape will be such that it can fit comfortably around the eye and the configuration of the body will be such that it can readily be gripped with the thumb and first finger of a hand of a user of the applicator.

### Brief Description of the Drawings

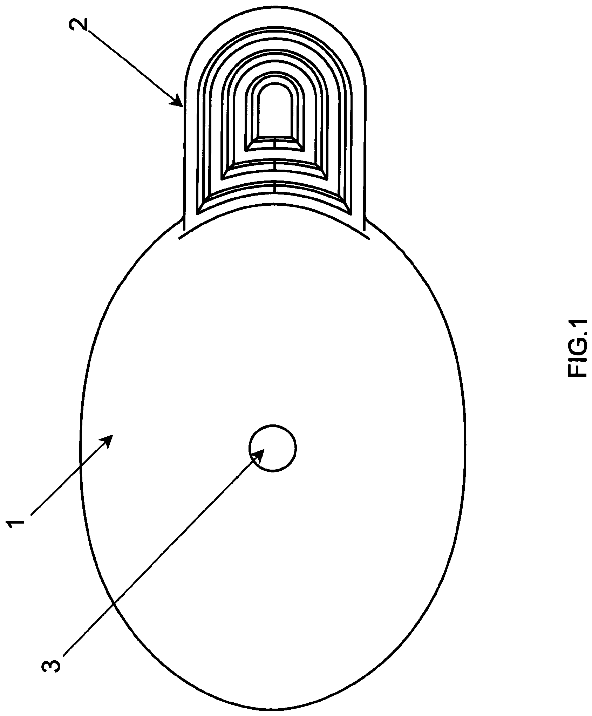

Figure 1 is a plan view of the eye drop applicator,

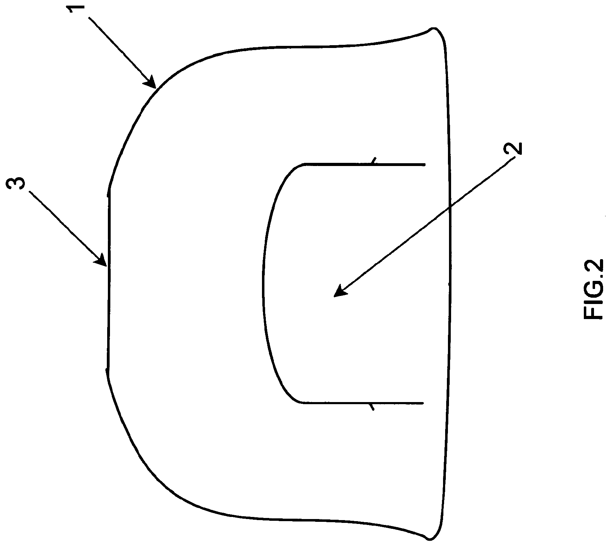

Figure 2 is a front view of the applicator,

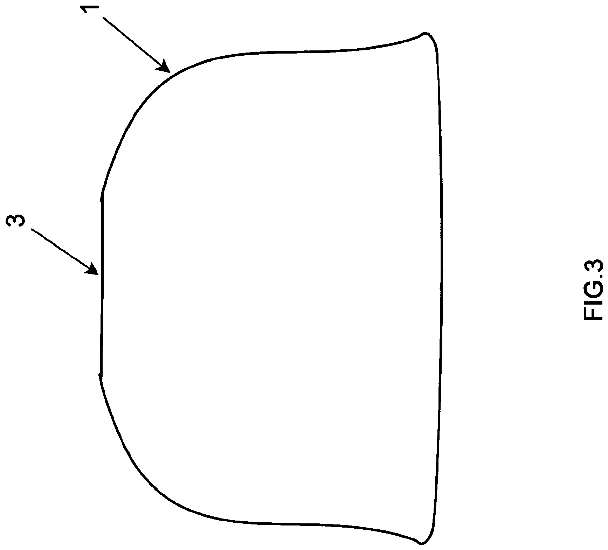

Figure 3 is a rear view of the applicator,

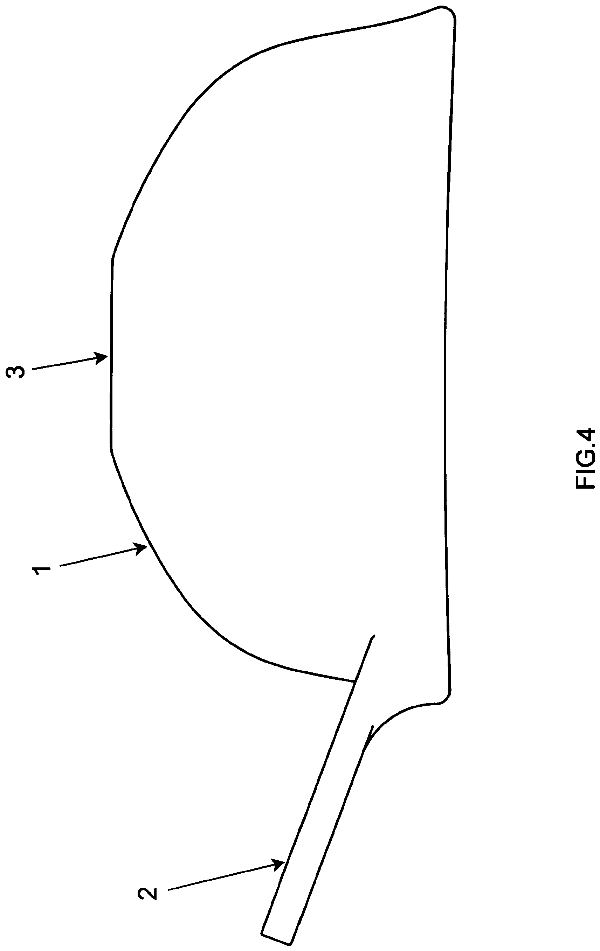

Figure 4 is a side view of the applicator, and

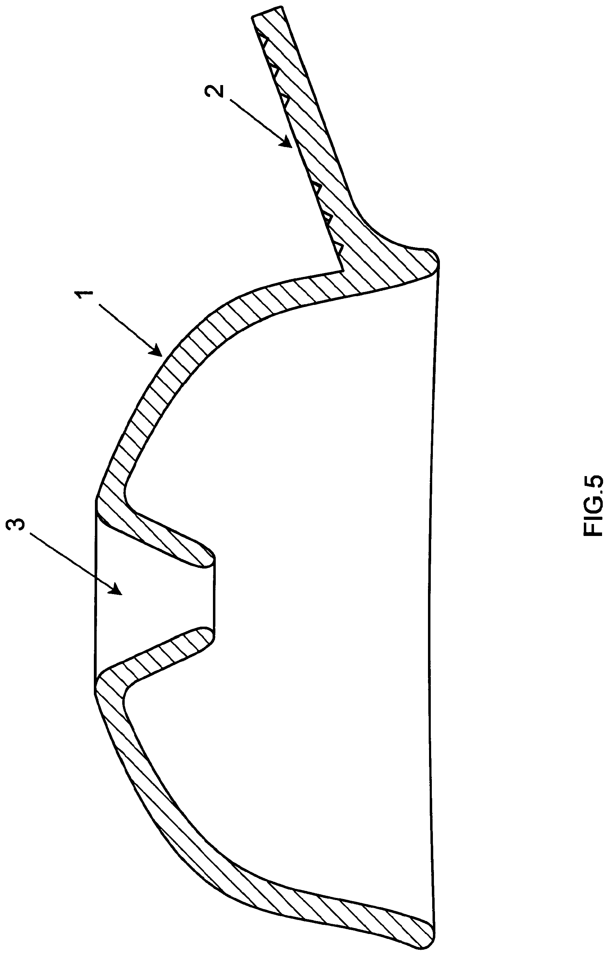

Figure 5 is a sectional view of the applicator.

### Description of the Preferred Embodiments

The eye drop applicator shown in the drawings is formed from a resiliently deformable plastics material which is either black in colour or a suitable dark shade so that it does not allow any light transmission. It includes a main body 1 having a shape similar to that of an inverted bowl but with an elliptical cross section so that it is easy to grasp in one hand when dispensing an eye drop. There is a central tapering opening 3 at the top of the application and this enables a variety of sizes of eye drop bottles to be inserted into the top of the applicator.

The applicator can be produced by a moulding procedure using a suitable resiliently deformable opaque material.

Before being used, the applicator should first be washed to ensure that it does not carry any dirt. If the user is going to insert an eye drop in his or her own eye, the main body 1 of the applicator is gripped between the first finger and the thumb of one hand with the palm of the hand uppermost. The applicator is then turned over and placed over the eye to be treated and pressed gently into position over the eye so as to ensure that there is no entry of light between the periphery of the applicator and the skin of the user.

With the other hand, the user takes a bottle of eye drops, removes any stopper and places the top of the bottle in the opening 3 at the top of the applicator. The user can see the bottle with vision from the other eye allowing him or her to place the top of the bottle of eye drops accurately and easily in the required position. The eye drop bottle is then squeezed gently so that a drop of the eye drop fluid falls downwardly through the opening 3 and is directed into the centre of the eye. After the eye drop has been applied, the applicator is removed and the user can then use a tissue to wipe the eye and the surrounding area to remove any excess fluid.

The eye drop applicator, according to the invention, is placed in position using the handle 2, which is grasped between the first finger and thumb, with the thumb on the underside of the handle 2 and the first finger on top of the handle 2. The application procedure is then carried out as described above, with the handle 2 facilitating secure placement of the applicator in the required position.

The provision of the small handle 2 makes it easier for a parent to hold the applicator in place when dispensing an eye drop to a child. It can also be used by a nurse when treating an elderly patient. The upper surface of the handle 2 can be patterned as shown so as to facilitate gripping thereof.

If the applicator is a "single use" device, it can be formed from paper or from a recycled and disposable pulp material with added waterproofing and manufactured in accordance with Medical Devices Directive 93/42/EEC. It will then, of course, not need to be washed before it is used.

## Claims

1. Use of an eye drop applicator which comprises a body (1) of generally inverted bowl shape formed of an opaque material with an opening (3) in the top of the inverted bowl into which the top of a bottle of eye drops can be inserted, **characterised by** the provision of a handle (2) projecting from the body (1) of bowl shape, the applicator being placed in position using the handle which is configured to be grasped by the first finger and thumb, with the thumb on the underside of the handle and the first finger on top of the handle.

2. Use of an eye drop applicator as claimed in Claim 1, in which the upper surface of the handle (2) is patterned so as to facilitate gripping thereof.

3. Use of an eye drop applicator as claimed in Claim 1 or 2, in which the opening (3) in the top of the inverted bowl is afforded by a tapering formation.

## Patentansprüche

1. Verwendung eines Augentropfen-Applikators, der einen Körper (1) von im Allgemeinen umgekehrter Schalenform umfasst, der aus einem undurchsichtigen Material mit einer Öffnung (3) in der Oberseite der umgekehrten Schale ausgebildet ist, in die die Oberseite einer Augentropfenflasche eingeführt werden kann, **gekennzeichnet durch** die Bereitstellung eines Griffs (2), der von dem schalenförmigen Körper (1) vorsteht, wobei der Applikator unter Verwendung des Griffs in Position gebracht wird, der dazu konfiguriert ist, mit Zeigefinger und Daumen gegriffen zu werden, wobei sich der Daumen an der Unterseite des Griffs und der Zeigefinger auf der Oberseite des Griffs befindet.

2. Verwendung eines Augentropfen-Applikators nach Anspruch 1, bei dem die obere Oberfläche des Griffs (2) gemustert ist, um ein Greifen desselben zu erleichtern.

3. Verwendung eines Augentropfen-Applikators nach Anspruch 1 oder 2, bei dem die Öffnung (3) in der Oberseite der umgekehrten Schale durch eine sich verjüngende Struktur erzeugt wird.

## Revendications

1. Utilisation d'un applicateur de gouttes ophtalmiques qui comprend un corps (1) en forme de bol généralement renversé formé d'un matériau opaque doté d'une ouverture (3) dans le haut du bol renversé dans laquelle le haut d'un flacon de gouttes ophtalmiques peut être inséré, **caractérisé par** la présence d'une poignée (2) faisant saillie du corps (1) en forme de bol, l'applicateur étant mis en place à l'aide de la poignée qui est conçue pour être saisie par l'index et le pouce, le pouce se trouvant sur la face inférieure de la poignée et l'index sur le haut de la poignée.

2. Utilisation d'un applicateur de gouttes ophtalmiques selon la revendication 1, ladite surface supérieure de la poignée (2) étant façonnée de façon à faciliter sa préhension.

3. Utilisation d'un applicateur de gouttes ophtalmiques selon la revendication 1 ou 2, ladite ouverture (3) dans le haut du bol inversé étant fournie par une formation conique.
